# EUROPÄISCHE PATENTSCHRIFT

(11) **EP 2 746 385 B1**
(45) Veröffentlichungstag und Bekanntmachung des Hinweises auf die Patenterteilung: **06.12.2017**
(21) Anmeldenummer: 13198623.4
(22) Anmeldetag: 19.12.2013
(51) Int. Cl.: C12M 1/107, C12M 1/00, A01C 3/02, F17C 1/00

(54) **Tragluftdach**
Air-supported roof
Plafond à double membrane

(30) Priorität: 21.12.2012 DE 102012112935; 21.12.2012 DE 202012105047 U
(43) Veröffentlichungstag der Anmeldung: 25.06.2014
(73) Patentinhaber: JOPE Beteiligungs GmbH, 87653 Eggenthal (DE)
(72) Erfinder: Baur, Josef, 87784 Westerheim (DE); Baur, Peter, 87724 Ottobeuren (DE)
(74) Vertreter: Schulz, Manfred

(56) Entgegenhaltungen:
- EP-A2- 1 338 843
- DE-A1- 4 120 986
- DE-A1- 10 142 108
- DE-U1-202011 000 577

## Beschreibung

Die Erfindung betrifft ein insbesondere kegel- oder kuppelförmiges Tragluftdach für einen Fermentationsbehälter einer Biogasanlage nach dem Oberbegriff des Patentanspruches 1.

Derartige kegel- oder kuppelförmige Tragluftdächer für einen Fermentationsbehälter einer Biogasanlage sind zahlreich aus dem Stand der Technik bekannt, wie zum Beispiel aus der AT 412116 B und der hierzu äquivalenten EP 1447613 B1. Dort ist ein Gasspeicher mit Tragluftdach offenbart, bei dem ein entlang der Innen- oder Außenseite der Aussenmembran sich erstreckender Zuführkanal mit einer Vielzahl von Gas-Eintrittslöchern zur Einleitung des Hilfsgases in den Zwischenraum vorgesehen ist, welcher Zuführkanal eingangsseitig mit der Zuführvorrichtung verbunden ist, wobei die Wand des Zuführkanals durch ein flexibles Flächenelement gebildet ist, das mit der Außenmembran verbunden ist und wobei die Anschlussöffnung für den Anschlussstutzen der Zuführvorrichtung im flexiblen Flächenelement vorgesehen ist.

Nachteil des Tragluftdaches der AT 412116 B bzw. EP 1447613 B1 ist, dass die Stützluft zwar durch eine Vielzahl von Gas-Eintrittslöchern in den Zwischenraum zwischen die Innen- und Aussenmembran eingeführt wird, jedoch nur in einem sehr begrenzten Bereich des flexiblen Zuführkanals zwischen zwei Längenlinien von ca. 36° Breite. Gegenüber einer einzigen Eintrittsöffnung ist diese Konstruktion zwar verbessert im Hinblick auf die Gefahr des Verschlusses des Gaseintrittes durch direkte Anlage der beiden Membranen im Bereich des Gaseintrittes, jedoch ist diese Gefahr des Verschlusses des Gaseintrittes immer noch vorhanden. Auch ist das Einbringen der Gaseintrittslöcher in die Außenmembran mit einem entsprechenden Aufwand verbunden. Grundsätzlich verschlechtern solche Einlassöffnungen auch die mechanische Stabilität der Außenmembran.

Die eingeblasene Tragluft führt zu einer Beabstandung der inneren Biogasfolie und der außenliegenden Schutzfolie, was günstig ist für die Isolation, weil das Gährgut, welches sich im Silagebhälter befindet, am effektivsten verarbeitet wird, wenn ein entsprechendes Temperaturniveau eingehalten wird, das gegebenenfalls auch durch zusätzliches Heizen konstant gehalten wird. Um den Energieaufwand für das Beheizen zu reduzieren, ist eine Isolation des Fermentationsbehälters und somit auch des Tragluftdaches günstig, was durch den zweilagigen Aufbau und den zwischen Biogasfolie und Schutzfolie gebildeten, mit Tragluft gefüllten Innenraum erreicht wird.

Fällt aber nun das Gebläse, welches die Tragluft in den Innenraum fördert, aus, so fällt die außenliegende Schutzfolie auf die Biogasfolie herab, die Isolation bricht zusammen. Die deutlich verschlechterte Isolation führt zu einem schnelleren Wärmeverlust im Fermentationsbehälter, der entweder durch höheres Heizen und damit verbundenen höheren Energiekosten kompensiert werden kann oder aber der Gährungsprozess läuft schlechter, das heißt ineffizienter ab und die Ausbeute von Biogas sinkt.

Aus der Druckschrift DE 41 20 986 A1 ist eine Abdeckung von oben offenen Behältern bekannt. Diese offene Abdeckung ist insbesondere für Güllegruben für die Biogaserzeugung vorgesehen und besitzt eine nach außen hin angeordnete bzw. außenliegende Folie sowie eine davon unabhängig, innerhalb eines Biogassammelraumes liegende Folie, welche den Biogassammelraum von einer zwischen den Folien befindlichen Zwischenkammer gasdicht abtrennt.

Des Weiteren ist aus der Druckschrift DE 101 42 108 A1 eine Vorrichtung zum Halten von Folien, ein Dach- oder Wandelement mit einer solchen Vorrichtung sowie ein Dach- oder eine Wand mit derartigen Dach- bzw. Wandelementen bekannt. Das Dachelement kann dabei aus einem im Dachsparren gehaltenen Folienkissen bestehen, das eine obere Lage aus einer Folie, eine mittlere Lage aus einer nächsten Folie und eine untere Lage aus einer weiteren Folie aufweist. Das Folienkissen ist zusätzlich mit einer vierten unteren Lage aus einer akustisch wirksamen, microperforierten Folie versehen. Diese microperforierte Folie wird durch mehrere parallel zueinander verlaufende Luftschläuche auf Abstand zu der ihr benachbarten unteren Folie des Folienkissens gehalten.

In der Druckschrift US 2003/0019515 A1 wird eine pneumatische Wandstruktur und ein Verfahren zum Herstellen und Aufstellen derselben offenbart. Dabei wird eine leichtgewichtige Wärmeisolierung durch eine zweischichtige Konfiguration der Wandstruktur erreicht, die eine Innenschicht und eine Außenschicht umfasst.

Des Weiteren ist aus der Europäischen Patentanmeldung EP 1 338 843 A2 ein Gassammelbehälter mit einer flexiblen Membran bekannt, der insbesondere zum Speichern von Biogas dient. Der Gassammelbehälter weist eine erste Membran zum Bilden einer Gassammelkammer und eine zweite Membran zum Bilden einer Druckkammer auf, die angrenzend zu der Sammelkammer vorgesehen ist. Des Weiteren ist auch eine dritte Membran vorgesehen, die zusammen mit der ersten und zweiten Membran die Druckkammer ausbildet.

Aus der internationalen Patentanmeldung WO 2010/136885 A1 ist eine Füllstandsanzeige bekannt. Diese Füllstandsanzeige ist für einen Membrangasbehälter vorgesehen, der aus wenigstens einer Sammelkammer zum Speichern eines Gases besteht und eine flexible Membran aufweist, die sich zwischen einer Leer- und einer Vollposition des Membrangasbehälters bewegen kann. Die flexible Membran ist dabei über ein flexibles bzw. elastisches Mittel mit einer Außenfolie des Membrangasbehälters verbunden, welcher den Abstand zwischen den beiden Folien zu signalisieren in der Lage ist.

Das Dokument DE 41 20 986 A1 offenbart eine Behälterabdeckung zur konstruktiv einfachen Herstellung von Reaktionsbehältern, insbesondere von Biogasbehältern und auch zur Nachrüstung von oben offenen Behältern. Die offene Oberseite des Behälters ist mittels mindestens zwei übereinander angeordneten Folien abgedeckt, welche rundum am Grubenrand gasdicht befestigt sind und von denen die obere, nach außen hin liegende Folie unter einem konstanten, mehr als 1 at betragenden Gasdruck steht, der die Folie konvex nach außen steif aufwölbt. Zwischen der nach außen hin liegenden Folie und der innerhalb des Gasraums der Biogasgrube angeordneten Folie ist eine gesonderte Zwischenkammer vorgesehen, deren Gasvolumen kontrolliert veränderlich ist. Dabei sind die umfangsseitigen Randbereiche der nach außen hin liegenden Folie und der nach innen hin angeordneten Folie gemeinsam aneinander anliegend auf einer Schutzunterlage (z. B. Schutzfolie) mindestens zweifach gegen abgewinkelte Dichtkanten (Oberrand und Winkelprofile) angepresst, wobei sie mindestens an einer Dichtkante mittels eines zusätzlichen Spannrings über die Dichtkante zusammengepresst sind.

Es ist daher Aufgabe der vorliegenden Erfindung, die Wärmedämmung bei gattungsgemäßen Tragluftdächern einerseits, aber auch bei entsprechenden Fermentationsbehältern oder Biogasanlagen andererseits weiter zu verbessern. Aufgabe der Erfindung ist es auch, den fertigungstechnischen Aufwand für die Wärmedämmung zu reduzieren.

Zur Lösung der gestellten Aufgabe dienen die Merkmale des unabhängigen Anspruches 1, wobei vorteilhafte Weiterbildungen Gegenstand der abhängigen Ansprüche sind.

Wesentliches Merkmal der Erfindung ist, dass innerhalb des Innenraumes der Tragluft, zwischen Schutzfolie und Biogasfolie, mindestens ein, bevorzugt eine Vielzahl von Abstandshaltern vorgesehen ist/sind und der oder die Abstandshalter auf der dem Innenraum zugewandten Seite der Schutzfolie oder Biogasfolie, oder in in der Folie vorgesehenen Haltetaschen angeordnet ist/- sind.

Vorteil dieser Maßnahme ist, dass hierdurch unabhängig von Ort und Anzahl der Eintrittsöffnungen für die Tragluft des Tragluftdaches, die Schutzfolie und Biogasfolie immer auf Abstand gehalten werden. Die erfindungsgemäß vorgesehene Anordnung von einem beziehungsweise einer Vielzahl von Abstandshaltern bewirkt einen Mindestabstand zwischen der innenliegenden Biogasfolie und der außenliegenden Schutzfolie, wodurch zumindest auch bei einem Ausfall des Gebläses eine ausreichende Beabstandung zwischen diesen beiden Folien sichergestellt ist. Somit verbleibt noch ein Mindestrest an Isolation, die die Gesamteffizienz der Biogasanlage, die mit dem erfindungsgemäßen Tragluftdach ausgestattet ist, steigert.

Im Gegensatz zu allen eingangs erwähnten Lösungen des Standes der Technik wird durch die erfindungsgemäße Lösung erreicht, dass der fertigungstechnische Aufwand für die Herstellung einer funktionierenden Wärmedämmung reduziert wird. Dabei ist es gegeben, dass auch bei einem Ausfall der Tragluftbelüftung die Wärmedämmung weiterhin gewährleistet ist und zwar so, dass keine erhöhten Wärmeverluste durch den Ausfall der Tragluftkonstruktion zu verzeichnen sind. In geschickter Weise löst die Erfindung die Aufgabe dadurch, dass mindestens ein Abstandshalter, bevorzugt eine Vielzahl von Abstandhaltern innerhalb des Innenraumes der Tragluft zwischen Schutzfolie und Biogasfolie vorgesehen ist, und der oder die Abstandhalter auf der dem Innenraum zugewandten Seite der Schutzfolie oder der Biogasfolie, oder in in der Schutzfolie oder in in der Biogasfolie vorgesehenen Haltetaschen angeordnet ist/sind. Dies lässt sich fertigungstechnisch sehr schön herstellen, was später noch beschrieben wird. Des Weiteren besitzt diese Ausgestaltung natürlich den Vorteil, dass bei einem Ausfall der Tragluftzufuhr die beiden Folien nicht aufeinander zum Aufliegen kommen. Im Stand der Technik sind hierzu beispielsweise sehr aufwendige Wärmeschutzkonstruktionen vorgesehen, so ist zum Beispiel eine mehrlagige Folienkonstruktion für die Außenfolie vorgesehen, um die Wärmedämmung zu verbessern. Dies führt allerdings nicht unbedingt dazu, dass bei einem Ausfall der Tragluftzufuhr diese Konstruktion weiterhin zuverlässig den Innenraum vor Wärmeverlusten schützt. Vielmehr ist das Gegenteil der Fall. Auch zeichnen sich die weiteren eingangs erwähnten Lösungen des Standes der Technik durch eine konstruktiv sehr komplizierte Ausgestaltung aus, die natürlich den Aufwand zur Herstellung in erheblichem Maße erhöht. Durch die erfindungsgemäße Lösung werden diese aufwändigen Konstruktionen nicht mehr benötigt. Die Lösung schlägt demnach eine sichere Konstruktion vor, die sich in einfacher Weise herstellen lässt, insbesondere wesentlich einfacher als die eingangs beschriebenen Lösungen des Standes der Technik.

Der Abstandshalter kann dabei als einzelnes Stück oder Schlauch beziehungsweise profilartiges Element ausgebildet sein. Die erfindungsgemäß vorgeschlagenen Abstandshalter sind sehr variabel ausbildbar.

Bevorzugt wird, wenn die Abstandshalter innerhalb des Innenraumes der Tragluft regelmäßig verteilt angeordnet sind, insbesondere schachbrett-, raster-, oder matrixförmig. Hierdurch können die Folien sehr kostengünstig hergestellt werden, da die Abstandshalter unabhängig von der räumlichen Anordnung des oder der Tragluftgebläse, sowie der übrigen peripheren Vorrichtungen wie zum Beispiel Ein- und Ausfüll-, sowie Rührstutzen für die Silage und das Biogas, angeordnet werden können. Auch können Teile aus den Folien bei Reparaturen oder nachträglichen Änderungen der Peripherie einfach ausgeschnitten oder eingesetzt werden.

Besonders günstig ist, dass die Abstandshalter auf Längen- und/oder Breitengraden eines sphärisch oder kegelig ausgebildeten Tragluftdaches angeordnet sind, dessen Bereich des Pols bzw. der Kegelspitze frei von Abstandshaltern ist. Hierdurch wird zum einen eine einfache und kostengünstige Herstellung des Tragluftdaches ermöglicht. Zudem wird eine auf Explosionskonzentration (ca. 1-10 Vol-%) unerwünschte Anreicherung der Tragluft durch Biogas (zum Beispiel Methan), das aus der Silage durch die Biogasmembran hindurch diffundiert ist, im Bereich des Pols der Schutzmembran vermieden, da dort wegen des Fehlens der Abstandshalter eine optimale Verdünnung des Biogases mit als Spülluft wirkende, eingeblasene Tragluft erfolgt.

Von besonderem Vorteil ist es auch, wenn der oder die Abstandhalter überdeckungsfrei an oder in der Schutzfolie oder in der Biogasfolie angeordnet ist/sind. Eine solche Ausgestaltung hat natürlich den Vorteil, dass die Abstandhalter gasdicht noch von dem Innenraum getrennt sind und somit keine ungewünschten Diffusionen und/oder Durchgänge von Biogas in die Abstandshalter bzw. die Räume der Abstandshalter oder in die Taschen, in den sich die Abstandhalter befinden, vorkommen können.

Für die Anordnung der Abstandshalter gibt es eine Reihe von Möglichkeiten, so können diese sich auf der Schutzfolie oder auf der Biogasfolie befinden, oder aber auf der Schutzfolie und auf der Biogasfolie. Weiterhin können sich die Abstandshalter alternativ oder zusätzlich dazu auf einer kegel- oder kuppelförmigen mindestens für Biogas und Luft durchlässigen oder aber gasdichten Abstandshalter-Folie zwischen der Schutzfolie und der Biogasfolie befinden. Bevorzugt wird aber die Anordnung der Abstandshalter auf der Schutzfolie, da diese robust genug ist, diese aufzunehmen und keine Gefahr für die Biogasfolie besteht, gasundicht zu werden, auf Grund der Befestigung der Abstandshalter auf der Biogasfolie. Eine elegante alternative Lösung wäre, die Abstandshalter auf einer von der Schutzfolie und auch der Biogasfolie getrennten Abstandshalter-Folie anzuordnen, die dann zwischen Schutzfolie und Biogasfolie in das Tragluftdach integriert wird. Damit wären alle drei Funktionen getrennt voneinander in drei unterschiedlichen Folien verwirklicht, nämlich die Schutzfunktion in der äußeren relativ dicken Schutzfolie, die Gasdichtheit für Biogas in der dünnen inneren Biogasfolie, sowie die Abstandshaltefunktion in der dazwischen befindlichen Abstandshalter-Folie, die weder besonders robust, noch besonders gasdicht sein muss, sondern lediglich die Abstandshalter tragen muss. Alle diese drei Möglichkeiten der Anordnung der Abstandshalter auf den Folien können natürlich innerhalb eines einzigen Tragluftdaches kombiniert sein, oder aber auch nur zwei der drei Varianten.

Die Verwendung einer Abstandshalter-Folie hat den Vorteil, dass diese zum Beispiel auch aus dem gleichen Grundmaterial bestehen kann, wie die Schutzfolie beziehungsweise die Biogasfolie, ohne aber hierauf diesen Vorschlag beschränken zu wollen. Allgemein ist es möglich, anstelle einer Abstandshalter-Folie, auch eine Abstandshalter-Tragstruktur einzusetzen, die zum Beispiel netzartig oder aus einer Vielzahl von an Schnüren angeordneten Abstandshaltern bestehen kann.

Um die Montage des Tragluftdaches zu vereinfachen und damit kostengünstiger zu gestalten, kann es in einer vorteilhaften Ausführungsform vorgesehen sein, dass die (zum Beispiel kegel- oder kuppelförmige) Abstandshalter-Folie mit den darauf befindlichen Abstandshaltern mit der Schutzfolie und/oder der Biogasfolie verbunden ist. Damit wäre zwar entweder die Schutzfolie oder die Biogasfolie oder aber beide Folien jeweils aus zwei gleichen oder unterschiedlichen Folien aufgebaut, jedoch wäre damit ein Aufbauen des Tragluftdaches vor Ort wesentlich einfacher, da ja das Verbinden der Schutzfolie und/oder der Biogasfolie mit der Abstandshalter-Folie beim Hersteller der Folien geschehen würde.

Das Verbinden der Abstandshalter mit den Folien kann im Rahmen der vorliegenden Erfindung ebenfalls vielfältig erfolgen. So können die Abstandshalter mit den Folien (Schutzfolie und/oder der Biogasfolie und/oder Abstandshalter-Folie) unmittelbar oder mittelbar kraftschlüssig und/oder formschlüssig und/oder stoffschlüssig verbunden sein. Je nach Anwendungsfall kann dann die geeignete Art und Weise der Befestigung der Abstandshalter an den Folien erfolgen. Natürlich können zusätzlich oder alternativ die Abstandshalter auch integral aus dem Material der Folien selbst gebildet sein.

Insbesondere sind dabei die Abstandshalter mit den Folien (Schutzfolie und/oder der Biogasfolie und/oder Abstandshalter-Folie) verklebt, verschweißt, aufvulkanisiert, oder aufgenäht oder befinden sich in darauf verklebten, verschweißten, aufvulkanisierten oder aufgenähten Haltetaschen. Je nach Anwendungsfall kann dann die geeignete Art und Weise der Befestigung der Abstandshalter an den Folien erfolgen. Natürlich können auch separate oder aus dem Material der Folien selbst gebildete Haltetaschen selbst als Abstandshalter wirken, ohne dass dabei separate Abstandshalter in die Haltetaschen eingebracht werden müssen.

Je nach Größe und Form des Tragluftdaches können die Abstandshalter, aber auch deren Haltetaschen, (teil-)kugel-, streifen-, schlauch- oder vollzylinderförmig ausgebildet sein.

Damit der Abstand im Bereich zwischen 1 bis 20 cm, bevorzugt 5 bis 15 cm zwischen der Schutzfolie und der Biogasfolie gebildet werden kann, weisen die Abstandshalter etwa eine Höhe im Bereich zwischen 1 bis 20 cm, bevorzugt 5 bis 15 cm auf, ebenso deren Haltetaschen, falls vorhanden.

Als besonders bevorzugte Ausführungsform der vorliegenden Erfindung ist eine Vielzahl von paarweise angeordneten streifenförmigen Abstandshaltern vorgesehen, welche sich im Wesentlichen vom unteren Rand der Folien (Schutzfolie und/oder der Biogasfolie und/oder Abstandshalter-Folie) in deren Bereich des Poles erstrecken und paarweise zwischen sich Paar-Luftkanäle bilden, wobei am unteren Rand der Folien (Schutzfolie und/oder der Biogasfolie und/oder Abstandshalter-Folie) ein ringsumlaufender Ring-Luftkanal vorgesehen ist, welcher mit den Paar-Luftkanälen luftschlüssig verbunden oder verbindbar ist. Damit ist für jede Wetterlage auch bei starkem Wind und/oder Niederschlag dafür gesorgt, dass die Schutzfolie und Biogasfolie nicht direkt aufeinander zu liegen kommen und eine Tragluftzufuhr nicht mehr möglich wäre. Auch genügt deswegen auch nur eine einzige Zuführung der Tragluft über ein einziges Gebläse, da durch den Ring-Luftkanal am unteren Rand der Folien dafür gesorgt ist, dass die Tragluft auch zuverlässig in viele oder gar alle davon gespeisten Paar-Luftkanäle gelangt und damit die ideale Kegel- oder Kuppelform des Tragluftdaches während des bestimmungsgemäßen Gebrauches stets aufrecht erhalten bleibt. Durch den ringförmig umlaufend ausgebildeten Kanal gelangt die Tragluft über die paarweise angeordneten Abstandhalter dann natürlich zum Zentrum des Kuppeldaches nach oben und es ist gewährleistet, dass hier eine ständige Tragluftzufuhr über alle Kanäle zwischen zwei Abstandhaltern gewährleistet ist. Eine solche Konstruktion besitzt eine ausgezeichnete Stabilität auch gegenüber Belastungen, die von außen auf das Kuppeldach einwirken. So können hier besondere Schneelasten und/oder Sturm eine solchen Konstruktion nichts anhaben. Auch wenn die Zufuhr von Tragluft einmal ausbleiben sollte, fällt das Kuppeldach nicht gleich in sich zusammen, sondern wird noch ausreichend durch die Abstandhalter, die ja sternenförmig zum Zentrum hin verlaufen, gestützt.

Damit möglichst wenig Wärme aus der Silage bzw. aus dem durch deren Fermentation entstehenden Biogas entweicht, sind die Abstandshalter thermisch isolierendend und/oder thermisch isoliert ausgeführt. Insbesondere sind dabei die Abstandshalter und/oder deren Haltetaschen aus insbesondere geschäumtem Kunststoff ausgebildet. Als Werkstoffe kommen folgende Kunststoffe in Frage: Glasfaserverstärkte Kunststoffe (GFK), Kohlefaserverstärkte Kunststoffe (KFK), Polyethylen (PE), Polypropylen (PP), Polyvinylchlorid (PVC), Polystyrol (PS), Polyurethan (PU), Polyethylenterephtalat (PET), Polytertafluorethylen (PTFE), Polymethylmethacrylat (PMMA), oder eine beliebigen Kombination aus diesen Kunststoffen. Auch für die Folien können diese Kunststoffe verwendet werden, die aber dann in aller Regel nicht geschäumt sind.

Im Rahmen der Erfindung soll nicht nur das erfindungsgemäße Tragluftdach einer Biogasanlage geschützt werden, sondern durch einen selbständigen Nebenanspruch auch ein Fermentationsbehälter mit diesem erfindungsgemäßen kegel- oder kuppelförmigem Tragluftdach einer Biogasanlage.

Im Rahmen der Erfindung soll nicht nur das erfindungsgemäße Tragluftdach einer Biogasanlage geschützt werden, sondern durch einen selbständigen Nebenanspruch auch eine Folie (Schutzfolie und/oder der Biogasfolie und/oder Abstandshalter-Folie) für ein kegel- oder kuppelförmiges Tragluftdach für einen Fermentationsbehälter einer Biogasanlage.

In diesem Zusammenhang wird darauf hingewiesen, dass Merkmale, die im Zusammenhang mit dem Tragluftdach beschrieben und offenbart sind, in gleicher Weise auch für die Verwendung einer Folie für ein Tragluftdach oder einen Fermentationsbehälter in gleicher Weise als mitoffenbart und beschrieben anzusehen sind. Gegenstände und Merkmale, die nur in Bezug auf den Fermentationsbehälter beschrieben sind, gelten in gleicher Weise auch für das Tragluftdach beziehungsweise die Verwendung einer Folie als mitoffenbart. Merkmale, die nur im Zusammenhang mit der Verwendung der Folie offenbart sind, gelten in gleicher Weise auch für das Tragluftdach beziehungsweise den Fermentationsbehälter als mitoffenbart. Dem Fachmann ist die diesbezügliche Anordnung der Merkmale klar.

In den Zeichnungen ist die Erfindung in mehreren Ausführungsbeispielen schematisch dargestellt. Es zeigen:
- Figur 1a: Schematische Darstellung einer Seitenansicht im Schnitt eines erfindungsgemäßen Tragluftdaches auf einem Silagebehälter eines Fermentationsbehälters in einer ersten Ausführungsform mit Abstandshaltern auf der äußeren Schutzfolie;
- Figur 1b: Schematische Darstellung einer Seitenansicht im Schnitt eines erfindungsgemäßen Tragluftdaches auf einem Silagebehälter eines Fermentationsbehälters in einer zweiten Ausführungsform mit Abstandshaltern auf der inneren Biogasfolie;
- Figur 1c: Schematische Darstellung einer Seitenansicht im Schnitt eines erfindungsgemäßen Tragluftdaches auf einem Silagebehälter eines Fermentationsbehälters in einer dritten Ausführungsform mit Abstandshaltern auf der mittleren Abstandshalter-Folie;
- Figur 2: Ein Tragluftdach gemäß Figur 1 mit Darstellung von Varianten der Abstandshalter;
- Figur 3a: Schematische Darstellung im Schnitt durch die Abstandshalter tragende äußere Schutzfolie in einer ersten Ausführungsform;
- Figur 3b: Schematische Darstellung im Schnitt durch die Abstandshalter tragende äußere Schutzfolie in einer zweiten Ausführungsform;
- Figur 3c: Schematische Darstellung im Schnitt durch die Abstandshalter tragende äußere Schutzfolie in einer dritten Ausführungsform;
- Figur 4a: Schematische Darstellung im Schnitt durch die Abstandshalter tragende inneren Biogasfolie in einer ersten Ausführungsform;
- Figur 4b: Schematische Darstellung im Schnitt durch die Abstandshalter tragende inneren Biogasfolie in einer zweiten Ausführungsform;
- Figur 4c: Schematische Darstellung im Schnitt durch die Abstandshalter tragende inneren Biogasfolie in einer dritten Ausführungsform;
- Figur 5: Schematische Darstellung der Draufsicht im Schnitt eines erfindungsgemäßen Tragluftdaches auf einem Silagebehälter eines Fermentationsbehälters mit Ring-Luftkanal und sternförmig verlaufenden Paar-Luftkanälen.

In den Figuren sind gleiche oder einander entsprechende Elemente jeweils mit den gleichen Bezugszeichen bezeichnet und werden daher, sofern nicht zweckmäßig, nicht erneut beschrieben.

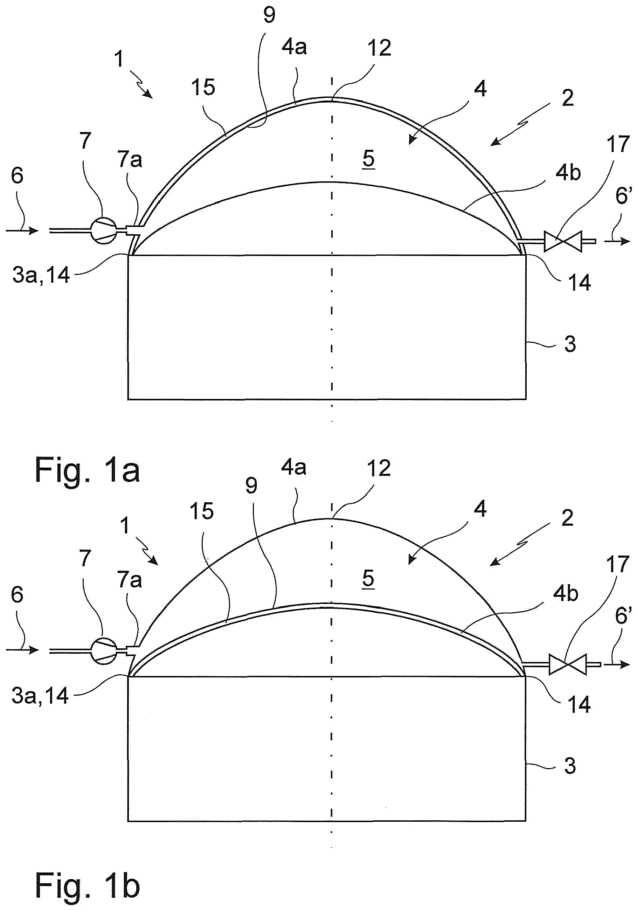

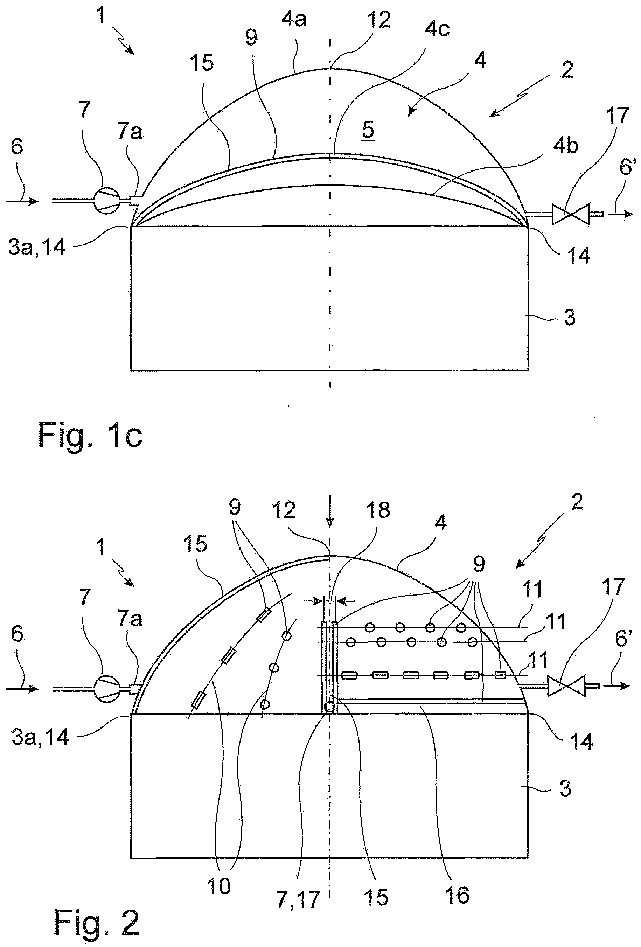

Figuren 1a, 1b und 1c zeigen eine schematische Darstellung einer Seitenansicht im Schnitt eines erfindungsgemäßen Tragluftdaches 2 mit Folien 4 auf dem oberen Rand 3a des Silagebehälters 3 eines Fermentationsbehälters 1 in drei Ausführungsformen, einmal mit Abstandshaltern 9 auf der äußeren Schutzfolie 4a (Fig.1a), einmal mit Abstandshaltern 9 auf der inneren Biogasfolie 4b (Fig.1b) und einmal mit Abstandshaltern 9 auf der mittleren Abstandshalter-Folie 4c (Fig. 1c).

In den Innenraum 5 zwischen die äußeren Schutzfolie 4a und die innere Biogasfolie 4b kann über den Anschlussstutzen 7a des Luftgebläses 7 Tragluft 6 eingeführt werden, um die äußere Schutzfolie 4a auf Abstand 8 zur inneren Biogasfolie 4b zu halten. Dabei sorgt mindestens ein Luft-Auslassventil 17 für die Ableitung eines geringen Anteils der Tragluft 6 aus dem Innenraum 5, um deren Konzentration von Biogas (zum Beispiel Methan) außerhalb des explosionsgefährdenden Bereichs von 1-10 Vol-% zu halten. Die an sich Biogas freie reine Tragluft 6 wird über das oder die Luftgebläse 7 in den Innenraum 5 gebracht, wo sie sich mit Biogas vermischt, das durch Fermentation der Silage aus dem Silagebehälter 3 durch die an sich gasdichte Biogasfolie 4b hindurch in den Innenraum 5 hinein diffundiert, so dass es nötig ist, die mit weniger als 1 Vol-% Biogas angereicherte Tragluft 6' über das oder die Luft-Auslassventil(e) 17 wieder aus dem Innenraum 5 (ins Freie) abzulassen, was dem Explosionsschutz dient. Bei ideal gasdichten inneren Biogasfolien 4b oder für Biogas leicht durchlässigen äußeren Schutzfolien 4a kann das Luft-Auslassventil 17 aber auch entfallen.

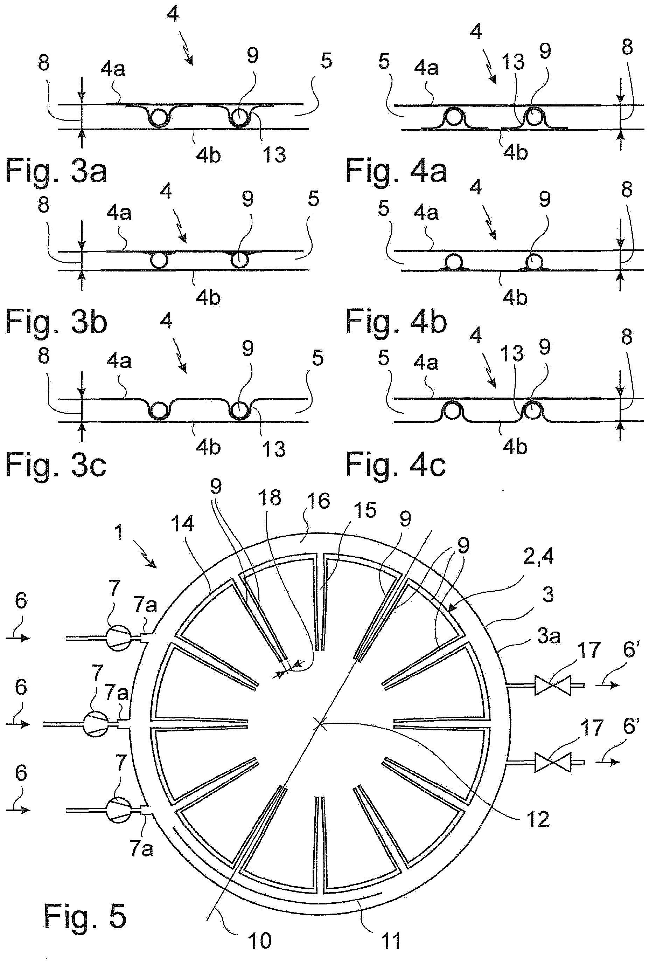

Wichtig für die vorliegende Erfindung ist aber, dass Abstandshalter 9 auf der äußeren Schutzfolie 4a (Fig. la) und/oder der inneren Biogasfolie 4b (Fig. 1b) und/oder der mittleren Abstandshalter-Folie 4c (Fig. 1c) vorgesehen sind, die in Figuren 1a, 1b und 1c ähnlich wie in Figur 5 zwischen sich Paar-Luftkanäle 15 bilden. Im Unterschied zu Figur 5 erstrecken sich unterbrechungslos die Paar-Luftkanäle 15 der Figuren 1a, 1b und 1c auf Längengraden 10 vom unteren Rand 14 der Folien 4 über den Pol 12 wieder zum unteren Rand 14 der Folien 4, so dass unterbrechungslose Paare von Abstandshaltern 9 vorhanden sind. In Figur 5 hingegen erstrecken sich die Paar-Luftkanäle 15 auf Längengraden 10 vom unteren Rand 14 der Folien 4 in Richtung Pol 12, jedoch enden diese vor dem Bereich des Pols 12, so dass keine unterbrechungslose Paare von Abstandshaltern 9 vorhanden sind. In Figur 2 sind beide Varianten der Abstandshaltern 9 gemäß den Figuren la und 5 gezeichnet.

Die Paare von Abstandshaltern 9 bilden in Figur 2 und 5 durch geringen Abstand 18 zwischen sich den Paar-Luftkanal 15, durch welchen hindurch dann die Tragluft 6, 6' über die Anschlussstutzen 7a des Luftgebläses 7 und der Luft-Auslassventile 17 strömen kann, wenn die äußere Schutzfolie 4a auf der inneren Biogasfolie 4b unerwünscht aufliegt. So ist stets dafür gesorgt, dass sich Tragluft 6, 6' zwischen der äußeren Schutzfolie 4a und der inneren Biogasfolie 4b befindet und diese nicht direkt aufeinander liegen. Bei direktem Kontakt zwischen äußerer Schutzfolie 4a und innerer Biogasfolie 4b besteht nämlich die Gefahr, dass zum einen die Tragluft 6, 6' nicht mehr in den Innenraum 5 gefördert werden kann und das Tragluftdach 2 seine Form verliert und schlimmstenfalls in sich zusammenfällt, und zum anderen Wärmebrücken entstehen, über welche die Wärme des Biogases durch die Biogasfolie 4b, gegebenenfalls die Abstandshalter-Folie 4c und die Schutzfolie 4a ins Freie gelangt, was den thermischen Wirkungsgrad der Biogasanlage verringert. Durch die erfindungsgemäßen Abstandshalter 9 wird daher im bestimmungsgemäßen Gebrauch des Fermentationsbehälters 1 immer dafür gesorgt, dass der thermische Wirkungsgrad der Biogasanlage maximal erhalten bleibt. Die durch die Paare von Abstandhaltern 9 gebildeten Paar-Luftkanäle 15 sind auch ausreichend stabil, um bei einem Ausfall der Tragluftzufuhr zumindest über eine gewisse Zeitdauer das Kuppeldach des Fermentationsbehälters 1 in seiner Form zu halten und insbesondere zu verhindern, dass ungewollt Wärme aus der Biogasanlage entweichen kann. Als besonders geschickt ist dabei die Ausgestaltung zu betrachten, wonach ein umlaufender Ringluftkanal 16 dafür sorgt, dass alle Paare von Abstandhaltern bzw. die dazwischen gebildeten Paar-Luftkanäle 15 ausreichend mit Tragluft versorgt werden und diese Tragluft in Richtung der Kuppel 12 transportiert wird.

In Figur 2 ist noch dargestellt, dass die Abstandshalter 9 punkt-, oder streifenförmig ausgebildet sein können und auf Längengraden 10 und/oder Breitengraden 11 der kugelförmigen Kuppel des Tragluftdaches 2 angeordnet sein können. Die Abstandshalter 9 können daher beliebig in Form einer Matrix auf den Folien 4 des Tragluftdaches 2 möglichst regelmäßig verteilt sein, wenn auch in gewissem Maße von dieser Regelverteilung abgewichen werden kann, falls Platz auf den Folien 4 für die Einlass- oder Auslassstutzen des Luftgebläses 7, der Luft-Auslassventile 17 oder eines (nicht dargestellten) Rührwerkes für die Silage oder einer (nicht dargestellten) Absaugpumpe zur Absaugung des Biogases zwischen innerer Biogasfolie 4b und Oberkante des Silagebehälters 3 benötigt werden sollte.

In Figur 2 ist auch ein Ring-Luftkanal 16 auf einem Breitengrad 11 im Bereich des unteren Randes 14 der Folien 4 dargestellt. Der Ring-Luftkanal 16 ist luftschlüssig mit einem auf einem Längengrad 10 befindlichen Paar-Luftkanal 15 verbunden, sowie mit dem Luftgebläse 7 und dem Luft-Auslassventil 17. Sowohl der Ring-Luftkanal 16, als auch der Paar-Luftkanal 15 sind durch zwei zugeordnete voneinander durch den geringen Abstand 18 getrennte parallele oder auf unterschiedlichen Breitengraden 10 (Fig. 5) befindlichen streifenförmigen Abstandshaltern 9 gebildet. Die streifenförmigen Abstandshalter 9 in Figur 2 können vor dem Bereich des Pols 12 enden oder aber ununterbrochen über den Pol 12 gehen und von Rand 14 zum gegenüberliegenden Rand 14 der Folien 4 reichen.

Figuren 3a-c und 4a-c zeigen nun schematisch den Aufbau der Folien 4 des Tragluftdaches 2 in sechs unterschiedlichen Varianten, wobei die hier zylinderförmigen Abstandshalter 9 in Figuren 3a-c im Innenraum 5 zwischen den Folien 4 an der äußeren Schutzfolie 4a angeordnet sind, wohingegen in Figuren 4a-c die hier zylinderförmigen Abstandshalter 9 im Innenraum 5 zwischen den Folien 4 an der inneren Biogasfolie 4b angeordnet sind. Die Abstandshalter 9 sorgen für einen stets vorhandenen minimalen Abstand 8 der Folien 4, der im Bereich zwischen 1-10 cm liegen kann, je nach Dimensionen des gesamten Tragluftdaches 2.

Figuren 3a und 4a zeigen, dass die Abstandshalter 9 in schlauchförmigen Haltetaschen 13 lose und/oder kraftschlüssig eingeführt sind, wobei die Haltetaschen 13 über herkömmliche Verbindungsmethoden an den Folien 4 formschlüssig und/oder stoffschlüssig festgelegt sind.

Figuren 3c und 4c zeigen, dass die Haltetaschen 13 für die Abstandshalter 9 gemäß Figuren 3a und 4a direkt aus dem Material der Folien 4 gebildet sind, wobei die Abstandshalter 9 dort über herkömmliche Verbindungsmethoden an den Folien 4 kraftschlüssig und/oder formschlüssig und/oder stoffschlüssig festgelegt sind.

Figuren 3b und 4b zeigen, dass die Abstandshalter 9 direkt auf die Oberfläche der Folien 4 über herkömmliche formschlüssige und/oder stoffschlüssige Verbindungsmethoden wie zum Beispiel Kleben, Schweißen, Vulkanisieren, Nähen aufgebracht sind.

Insgesamt wird damit ein gattungsgemäßes kegel- oder kuppelförmiges Tragluftdach 2 für einen Fermentationsbehälter 1 einer Biogasanlage derart weiter gebildet, dass die Öffnungen in den Membranen bzw. Folien für den Gas- bzw. Lufteintritt und gegebenenfalls -austritt zum Abstützen der Aussenfolie 4a im bestimmungsgemäßen Betrieb stets offen gehalten werden und dadurch eine länger andauernde Anlage der Membranen bzw. Folien 4 zuverlässig vermieden wird, wodurch der thermische Wirkungsgrad der Biogasanlage und damit der Ertrag an Wärmeenergie und elektrischer Energie konstant auf hohem Niveau gehalten werden kann.

### Der Vorschlag umfasst:

Ein Tragluftdach (2) für einen Fermentationsbehälter (1) einer Biogasanlage, welches mindestens zwei auf dem Rand (3a) eines Silagebehälters (3) möglichst gasdicht befestigte vertikal übereinander liegende Folien (4) beinhaltet, von denen mindestens eine als äußere Schutzfolie (4a) zum Schutz vor mechanischer Beschädigung mindestens einer möglichst gasdichten inneren Biogasfolie (4b) dient, wobei in den Innenraum (5) zwischen Schutzfolie (4a) und Biogasfolie (4b) Tragluft (6) über ein Luftgebläse (7) und einen Anschlussstutzen (7a) einführbar ist, welche die Schutzfolie (4a) stützt und von der Biogasfolie (4b) auf Abstand (8) haltbar beziehungsweise gehalten ist, wobei innerhalb des mit Tragluft (6) befüllbaren Innenraumes (5), zwischen Schutzfolie (4a) und Biogasfolie (4b), mindestens ein, bevorzugt eine Vielzahl von Abstandshaltern (9) vorgesehen ist/sind.

Ein Tragluftdach, wie zuvor ausgeführt, wobei die Abstandshalter (9) innerhalb des Innenraumes (5) regelmäßig verteilt angeordnet sind.

Ein Tragluftdach, wie zuvor ausgeführt, wobei die Abstandshalter (9) innerhalb des Innenraumes (5) der Tragluft (6) schachbrett-, raster-, oder matrixförmig angeordnet sind.

Ein Tragluftdach, wie zuvor ausgeführt, wobei die Abstandshalter (9) innerhalb des Innenraumes (5) der Tragluft (6) auf Längen- und/oder Breitengraden (10, 11) des sphärisch oder kegelig ausgebildeten Tragluftdaches (2) angeordnet sind, wobei insbesondere der Bereich des Pols (12) beziehungsweise der Kegelspitze frei von Abstandshaltern (9) ist.

Ein Tragluftdach, wie zuvor ausgeführt, wobei die Abstandshalter (9) sich auf der Schutzfolie (4a) und/oder auf der Biogasfolie (4b) befinden.

Ein Tragluftdach, wie zuvor ausgeführt, wobei sich die Abstandshalter (9) auf einer insbesondere flächigen Abstandshaltertragstruktur oder Abstandshalter-Folie (4c) zwischen der Schutzfolie (4a) und der Biogasfolie (4b) befinden.

Ein Tragluftdach, wie zuvor ausgeführt, wobei die Abstandshalterstruktur beziehungsweise Abstandshalter-Folie (4c) mit der Schutzfolie (4a) und/oder der Biogasfolie (4b), insbesondere in deren Randbereich (14), verbunden ist.

Ein Tragluftdach, wie zuvor ausgeführt, wobei die Abstandshalter (9) mit der Folie (4) unmittelbar oder mittelbar kraftschlüssig und/oder formschlüssig und/oder stoffschlüssig verbunden sind und/oder dass die Abstandshalter (9) integral aus dem Material der Folie (4) gebildet sind.

Ein Tragluftdach, wie zuvor ausgeführt, wobei die Abstandshalter (9) mit den Folien (4) verklebt, verschweißt, aufvulkanisiert, oder aufgenäht sind oder durch darauf verklebte, verschweißte, auf diese vulkanisierte, aufgenähte oder integral aus dem Material der Folie (4) gebildeten Haltetaschen (13) aufgenommen sind oder daraus selbst gebildet werden.

Ein Tragluftdach, wie zuvor ausgeführt, wobei die Haltetaschen (13) und/oder deren Abstandshalter (9) kugel-, teilkugel-, streifen-, schlauch- oder vollzylinderförmig ausgebildet sind.

Ein Tragluftdach, wie zuvor ausgeführt, wobei die Haltetaschen (13) schlauchförmig ausgebildet sind und insbesondere profilartige Abstandshalter (9) aufnehmen.

Ein Tragluftdach, wie zuvor ausgeführt, wobei die Abstandshalter (9) eine Höhe im Bereich zwischen 1 bis 20 cm, bevorzugt von 5 bis 15 cm aufweisen.

Ein Tragluftdach, wie zuvor ausgeführt, wobei mindestens ein, bevorzugt eine Vielzahl von paarweise angeordneten streifenförmigen Abstandshaltern (9) vorgesehen sind, welche sich im Wesentlichen vom unteren Rand (14) der Folien (4) in deren Bereich des Poles (12) erstrecken und paarweise zwischen sich Paar-Luftkanäle (15) bilden.

Ein Tragluftdach, wie zuvor ausgeführt, wobei am unteren Rand (14) der Folien (4) ein ringsumlaufender Ring-Luftkanal (16) vorgesehen ist, welcher mit den Paar-Luftkanälen (15) luftschlüssig verbunden oder verbindbar ist.

Ein Tragluftdach, wie zuvor ausgeführt, wobei der/die Abstandshalter (9) beziehungsweise mindestens ein Teil der Abstandshalter (9) im Randbereich (14) der Folie (4) angeordnet ist/sind.

Ein Tragluftdach, wie zuvor ausgeführt, wobei eine Anordnung der Abstandshalter (9) im Bereich des Anschlussstutzens (7a) und/oder eine Anordnung des Anschlussstutzens (7a) im Bereich des Ring-Luftkanals (16) beziehungsweise Paar-Luftkanal (15).

Ein Tragluftdach, wie zuvor ausgeführt, wobei die Abstandshalter (9) und/oder die Haltetaschen (13) thermisch isolierend und/oder isoliert ausgeführt sind.

Ein Tragluftdach, wie zuvor ausgeführt, wobei die Abstandshalter (9) aus insbesondere geschäumtem Kunststoff ausgebildet sind.

Ein Tragluftdach, wie zuvor ausgeführt, wobei die Folien (4) und/oder die Haltetaschen (13) und/oder die Abstandshalter (9) aus glasfaserverstärktem Kunststoff (GFK), kohlefaserverstärktem Kunststoff (KFK), Polyethylen (PE), Polypropylen (PP), Polyvinylchlorid (PVC), Polystyrol (PS), Polyurethan (PU), Polyethylenterephtalat (PET), Polytertafluorethylen (PTFE), Polymethylmethacrylat (PMMA), oder einer beliebigen Kombination daraus gebildet sind.

Ein Tragluftdach, wie zuvor ausgeführt, wobei das Tragluftdach (2) kegel- oder kuppelförmig ausgebildet ist.

Der Vorschlag betrifft auch einen Fermentationsbehälter einer Biogasanlage mit Tragluftdach (2), wobei das Tragluftdach (2) wie zuvor beschrieben ausgebildet ist.

Ebenfalls vom Vorschlag umfasst ist die Verwendung einer Folie (4) für ein insbesondere kegel- oder kuppelförmiges Tragluftdach (2) für einen Fermentationsbehälter (1) einer Biogasanlage, wobei die Folien (4) für das wie zuvor beschrieben ausgeführte insbesondere kegel- oder kuppelförmige Tragluftdach (2) ausgebildet sind.

Die im Zusammenhang mit den einzelnen Ausführungsformen des Vorschlags beschriebenen Merkmale, Teilmerkmale und/oder Merkmalskombinationen können selbstverständlich beliebig kombiniert werden, um den erfindungsgemäßen Gegenstand vorteilhaft weiterzubilden. Auch die Kombinationen von Merkmalen, Teilmerkmalen und/oder Merkmalskombinationen aus Ansprüchen verschiedener Anspruchskategorien sind gleichermaßen umfasst.

Die jetzt mit der Anmeldung und später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist von der Offenbarung dieser Anmeldung abgedeckt.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Tragluftdach (2) für einen Fermentationsbehälter (1) einer Biogasanlage, welches mindestens zwei auf dem Rand (3a) eines Silagebehälters (3) möglichst gasdicht befestigte vertikal übereinander liegende Folien (4) beinhaltet, von denen mindestens eine als äußere Schutzfolie (4a) zum Schutz vor mechanischer Beschädigung mindestens einer möglichst gasdichten inneren Biogasfolie (4b) dient, wobei in den Innenraum (5) zwischen Schutzfolie (4a) und Biogasfolie (4b) Tragluft (6) über ein Luftgebläse (7) und einen Anschlussstutzen (7a) einführbar ist, welche die Schutzfolie (4a) stützt und von der Biogasfolie (4b) auf Abstand (8) haltbar ist, wobei innerhalb des mit Tragluft (6) befüllbaren Innenraumes (5), zwischen Schutzfolie (4a) und Biogasfolie (4b), mindestens ein, bevorzugt eine Vielzahl von Abstandshaltern (9) vorgesehen ist/sind, **dadurch gekennzeichnet, dass** der oder die Abstandhalter (9) auf der dem Innenraum (5) zugewandten Seite der Schutzfolie (4a) oder der Biogasfolie (4b) oder in in der Schutzfolie (4a) oder Biogasfolie vorgesehenen Haltetaschen (13) angeordnet ist/sind.

2. Tragluftdach nach Anspruch 1, **dadurch gekennzeichnet, dass** die Abstandshalter (9) innerhalb des Innenraumes (5) regelmäßig verteilt angeordnet sind und/oder die Abstandshalter (9) innerhalb des Innenraumes (5) der Tragluft (6) Schachbrett-, raster-, oder matrixförmig angeordnet sind.

3. Tragluftdach nach Anspruch 1 oder 2, **dadurch gekennzeichnet, dass** die Abstandshalter (9) innerhalb des Innenraumes (5) der Tragluft (6) auf Längen- und/oder Breitengraden (10, 11) des sphärisch oder kegelig ausgebildeten Tragluftdaches (2) angeordnet sind, wobei insbesondere der Bereich des Pols (12) beziehungsweise der Kegelspitze frei von Abstandshaltern (9) ist.

4. Tragluftdach nach einem der Ansprüche 1 bis 3, **dadurch gekennzeichnet, dass** die Abstandshalter (9) sich auf der Schutzfolie (4a) und/oder auf der Biogasfolie (4b) befinden und/oder sich die Abstandshalter (9) auf einer insbesondere flächigen Abstandshaltertragstruktur oder Abstandshalter-Folie (4c) zwischen der Schutzfolie (4a) und der Biogasfolie (4b) befinden und/oder der oder die Abstandhalter (9) überdeckungsfrei an oder in der Schutzfolie (4a) oder der Biogasfolie (4b) angeordnet ist/sind.

5. Tragluftdach nach einem der Ansprüche 1 bis 4, **dadurch gekennzeichnet, dass** die Abstandshalterstruktur beziehungsweise Abstandshalter-Folie (4c) mit der Schutzfolie (4a) und/oder der Biogasfolie (4b), insbesondere in deren Randbereich (14), verbunden ist.

6. Tragluftdach nach einem der Ansprüche 1 bis 5, **dadurch gekennzeichnet, dass** die Abstandshalter (9) mit der Folie (4) unmittelbar oder mittelbar kraftschlüssig und/oder formschlüssig und/oder stoffschlüssig verbunden sind und/oder dass die Abstandshalter (9) integral aus dem Material der Folie (4) gebildet sind.

7. Tragluftdach nach einem der Ansprüche 1 bis 6, **dadurch gekennzeichnet, dass** die Abstandshalter (9) mit den Folien (4) verklebt, verschweißt, aufvulkanisiert, oder aufgenäht sind oder durch darauf verklebte, verschweißte, aufvulkanisierte, aufgenähte oder integral aus dem Material der Folie (4) gebildeten Haltetaschen (13) aufgenommen sind oder daraus selbst gebildet werden.

8. Tragluftdach nach einem der Ansprüche 1 bis 7, **dadurch gekennzeichnet, dass** die Haltetaschen (13) und/oder deren Abstandshalter (9) kugel-, teilkugel-, streifen-, schlauch- oder vollzylinderförmig ausgebildet sind und/- oder die Haltetaschen (13) schlauchförmig ausgebildet sind und insbesondere profilartige Abstandshalter (9) aufnehmen.

9. Tragluftdach nach einem der vorhergehenden Ansprüche, **dadurch gekennzeichnet, dass** die Abstandshalter (9) eine Höhe im Bereich zwischen 1 bis 20 cm, bevorzugt von 5 bis 15 cm aufweisen.

10. Tragluftdach nach einem der vorhergehenden Ansprüche, **dadurch gekennzeichnet, dass** mindestens ein, bevorzugt eine Vielzahl von paarweise angeordneten streifenförmigen Abstandshaltern (9) vorgesehen sind, welche sich im Wesentlichen vom unteren Rand (14) der Folien (4) in deren Bereich des Poles (12) erstrecken und paarweise zwischen sich Paar-Luftkanäle (15) bilden.

11. Tragluftdach nach einem der vorhergehenden Ansprüche, **dadurch gekennzeichnet, dass** am unteren Rand (14) der Folien (4) ein ringsumlaufender Ring-Luftkanal (16) vorgesehen ist, welcher mit den Paar-Luftkanälen (15) luftschlüssig verbunden oder verbindbar ist und/oder der/die Abstandshalter (9) beziehungsweise mindestens ein Teil der Abstandshalter (9) im Randbereich (14) der Folie (4) angeordnet ist/sind.

12. Tragluftdach nach einem der vorhergehenden Ansprüche, **gekennzeichnet durch** eine Anordnung der Abstandshalter (9) im Bereich des Anschlussstutzens (7a) und/oder eine Anordnung des Anschlussstutzens (7a) im Bereich des Ring-Luftkanals (16) beziehungsweise Paar-Luftkanal (15) und/- oder die Abstandshalter (9) und/oder die Haltetaschen (13) thermisch isolierend und/oder isoliert ausgeführt sind und/oder die Abstandshalter (9) aus insbesondere geschäumtem Kunststoff ausgebildet sind.

13. Tragluftdach nach einem der vorhergehenden Ansprüche, **dadurch gekennzeichnet, dass** die Folien (4) und/oder die Haltetaschen (13) und/oder die Abstandshalter (9) aus Glasfaserverstärktem Kunststoff (GFK), Kohlefaserverstärktem Kunststoff (KFK), Polyethylen (PE), Polypropylen (PP), Polyvinylchlorid (PVC), Polystyrol (PS), Polyurethan (PU), Polyethylenterephtalat (PET), Polytertafluorethylen (PTFE), Polymethylmethacrylat (PMMA), oder einer beliebigen Kombination daraus gebildet sind und/oder das Tragluftdach (2) kegel- oder kuppelförmig ausgebildet ist.

14. Fermentationsbehälter einer Biogasanlage mit Tragluftdach (2), **dadurch gekennzeichnet, dass** das Tragluftdach (2) nach einem der vorhergehenden Ansprüche ausgebildet ist.

15. Verwendung einer Folie (4) für ein insbesondere kegel- oder kuppelförmiges Tragluftdach (2) für einen Fermentationsbehälter (1) einer Biogasanlage, **dadurch gekennzeichnet, dass** die Folien (4) für das insbesondere kegel- oder kuppelförmige Tragluftdach (2) nach einem der Ansprüche 1 bis 13 ausgebildet sind.

## Claims

1. Air supported roof (2) for a fermentation tank (1) of a biogas plant, comprising at least two foils (4) vertically resting one above the other and attached as gas-proof as possible to the edge (3a) of a silage tank (3), at least one of them serving as outer protection foil (4a) for the protection against mechanic damage of at least one inner biogas foil (4b) as gas-proof as possible, wherein in the interior (5) between protection foil (4a) and biogas foil (4b) carrying air (6) can be introduced via an air blower (7) and a connection piece (7a), supporting the protection foil (4a) and keeping a distance (8) from the biogas foil (4b), wherein within the interior (5), that can be filled with carrying air (6), between protection foil (4a) and biogas foil (4b) at least one, preferably a multitude of spacers (9) is provided, **characterized in that** the spacer(s) (9) is/are arranged on the side of the protection foil (4a) or the biogas foil (4b) facing the interior (5), or in pockets (13) provided in the protection foil (4a) or the biogas foil.

2. Air supported roof according to claim 1, **characterized in that** the spacers (9) are arranged regularly spread within the interior (5), and/or the spacers (9) are arranged within the interior (5) of the carrying air (6) like a checker board, raster or matrix.

3. Air supported roof according to claims 1 or 2, **characterized in that** the spacers (9) are arranged within the interior (5) of the carrying air (6) on lines of longitude and/or latitude (10, 11) of the spherically or cone-like designed air supported roof (2), wherein in particular the area of the pole (12) or the cone tip is free of spacers (9).

4. Air supported roof according to one of the claims 1 to 3, **characterized in that** the spacers (9) are located on the protection foil (4a) and/or the biogas foil (4b), and/or the spacers (9) are located on an in particular two-dimensional spacer carrying structure or spacer foil (4c) between the protection foil (4a) and the biogas foil (4b), and/or the spacer(s) is/are arranged without overlapping at or in the protection foil (4a) or biogas foil (4b).

5. Air supported roof according to one of the claims 1 to 4, **characterized in that** the spacer structure or the spacer foil (4c) is connected with the protection foil (4a) and/or the biogas foil (4b), in particular at their fringe (14).

6. Air supported roof according to one of the claims 1 to 5, **characterized in that** the spacers (9) are connected with the foil (4) immediately or indirectly in a force-fitting and/or interlocking and/or material-locking manner, and/or the spacers (9) are formed integrally from the material of the foil (4) .

7. Air supported roof according to one of the claims 1 to 6, **characterized in that** the spacers (9) are glued, welded, cured, or sewed on the foils (4), or are received in or formed by pockets (13) glued, welded, cured, sewed thereon or integrally formed by the material of the foil (4).

8. Air supported roof according to one of the claims 1 to 7, **characterized in that** the pockets (13) and/or their spacers (9) are designed spherically, part-spherically, strip-, hose- or full cylinder-like, and/or the pockets (13) are designed hose-like and receive in particular profile-like spacers (9).

9. Air supported roof according to one of the preceding claims, **characterized in that** the spacers (9) have a height between 1 and 20 cm, preferably from 5 to 15 cm.

10. Air supported roof according to one of the preceding claims, **characterized in that** at least one, preferably a multitude of strip-like spacers (9) arranged in pairs is provided extending essentially from the bottom edge (14) of the foils (4) in the area of the pole (12), and form as pairs between each other pair air channels (15).

11. Air supported roof according to one of the preceding claims, **characterized in that** at the bottom edge (14) of the foils (4) a circular ring air channel (16) is provided connected or connectable by air with the pair air channels (15), and/or the spacer(s) (9) or at least a part of the spacers (9) is/are arranged at the fringe (14) of the foil (4).

12. Air supported roof according to one of the preceding claims, **characterized by** an arrangement of the spacers (9) in the area of the connection piece (7a), and/or an arrangement of the connection piece (7a) in the area of the ring air channel (16) or the pair air channel (15), and/or by a configuration with thermally insulating and/or insulating spacers (9) and/or pockets (13), and or a design with spacers (9) of in particular foamed plastic.

13. Air supported roof according to one of the preceding claims, **characterized in that** the foils (4) and/or the pockets (13) and/or the spacers (9) are formed from glass-fiber reinforced plastic (GFK), carbon-fiber reinforced plastic (KFK), polyethylene (PE), polypropylene (PP), polyvinyl chloride (PVC), polystyrene (PS), polyurethane (PU), polyethylene terephthalate (PET), polytetrafluorethylene (PTFE), polymethylmethacrylate (PMMA), or any combination thereof, and/or the air supported roof (2) is configured cone- or dome-shaped.

14. Fermentation tank of a biogas plant with air supported roof (2), **characterized in that** the air supported roof (2) is configured according to one of the preceding claims.

15. Use of a foil (4) for an in particular cone- or dome-shaped air supported roof (2) for a fermentation tank (1) of a biogas plant, **characterized in that** the foils (4) for the in particular cone- or dome-shaped air supported roof (2) are designed according to one of the claims 1 to 13.

## Revendications

1. Plafond à double membrane (2) destiné à un conteneur de fermentation (1) d'une installation biogaz, le plafond comportant au moins deux membranes (4) superposées verticalement et fixées de préférence de façon étanche au gaz au niveau du bord (3a) d'un silo d'ensilage (3), dont au moins une sert comme membrane de protection extérieure (4a) contre un endommagement mécanique et au moins une autre sert comme membrane intérieure étanche au gaz (4b) avec l'espace intérieur (5) entre la membrane de protection (4a) et la membrane de biogaz (4b) pouvant être rempli avec de l'air porteur (6) par une soufflerie d'air (7) via un manchon de raccordement (7a) supportant la membrane de protection (4a) et la maintenant à une distance (8) de la membrane de biogaz (4b) et avec au moins un, et de préférence plusieurs écarteurs (9) situé(s) à l'intérieur de l'espace intérieur (5) pouvant être rempli avec de l'air porteur (6) enfermé entre la membrane de protection (4a) et la membrane de biogaz (4b), **caractérisé en ce que** le ou les écarteur (s) (9) est (sont) situé (s) du côté intérieur (5) au niveau de la membrane de protection (4a) ou au niveau de la membrane de biogaz (4b) ou dans des poches de fixation prévues à l'intérieur de la membrane de protection (4a) ou à l'intérieur de la membrane de biogaz (4b).

2. Plafond à double membrane selon la revendication 1, **caractérisé en ce que** les écarteurs (9) sont distribués à distances égales dans l'espace intérieur (5) et/ou **en ce que** les écarteurs (9) sont distribués dans l'espace intérieur (5) rempli d'air porteur (6) selon des schémas de damier, de grille ou de matrice.

3. Plafond à double membrane selon la revendication 1 ou 2, **caractérisé en ce que** les écarteurs (9) sont situés dans l'espace intérieur (5) rempli d'air porteur (6) le long des lignes de longitude et/ou de latitude (10, 11) du plafond à double membrane sphérique ou conique (2) sauf en particulier dans la zone du pôle (12) ou de la pointe du cône qui reste sans écarteur (9).

4. Plafond à double membrane selon une des revendications 1 à 3, **caractérisé en ce que** les écarteurs (9) sont situés sur la membrane de protection (4a) et/ou sur la membrane de biogaz (4b) et/ou **en ce que** les écarteurs (9) sont situés au niveau d'une structure de support d'écarteurs ou au niveau d'une membrane d'écarteurs (4c) situés entre la membrane de protection (4a) et la membrane de biogaz (4b) et/ou **en ce que** le ou les écarteur(s) est(sont) disposé(s) sans recouvrement au niveau de la membrane de protection (4a) ou de la membrane de biogaz (4b) .

5. Plafond à double membrane selon une des revendications 1 à 4, **caractérisé en ce que** la structure de support d'écarteurs ou la membrane d'écarteurs (4c) est liée à la membrane de protection (4a) et/ou à la membrane de biogaz (4b) et ceci, en particulier au niveau de leur bord (14).

6. Plafond à double membrane selon une des revendications 1 à 5, **caractérisé en ce que** les écarteurs (9) interagissent directement ou indirectement avec la membrane (4) par la force de frottement et/ou par leur forme et/ou par liaison de matière et/ou **en ce que** les écarteurs (9) sont intégralement formés de la matière de la membrane (4).

7. Plafond à double membrane selon une des revendications 1 à 6, **caractérisé en ce que** les écarteurs (9) sont collés, soudés, vulcanisés ou cousus sur les membranes (4) ou **en ce qu'**ils sont logés dans des poches de fixation (13) collées, soudées, vulcanisées ou cousues sur les membranes (4) ou formées intégralement dans la matière des membranes (4) ou **en ce que** les écarteurs sont fabriqués directement dans la matière des membranes.

8. Plafond à double membrane selon une des revendications 1 à 7, **caractérisé en ce que** les poches de fixation (13) et/ou leur écarteur (9) possède une forme de sphère, d'une sphère partielle, d'une bande, d'un tube ou d'un cylindre plein et/ou **en ce que** les poches de fixation (13) ont une forme de tube et permettent d'y loger en particulier des écarteurs profilés (9) .

9. Plafond à double membrane selon une des revendications précédentes, **caractérisé en ce que** les écarteurs (9) possèdent une hauteur allant de 1 à 20 cm et de préférence de 5 à 15 cm.

10. Plafond à double membrane selon une des revendications précédentes, **caractérisé en ce qu'**au moins un, et de préférence plusieurs, écarteur(s) (9) est(sont) prévu(s) sous forme de bande et placé(s) par paire(s) allant de la zone de bordure inférieure (14) des membranes (4) vers la zone de pôle (12) en créant par paire des canaux d'air (15).

11. Plafond à double membrane selon une des revendications précédentes, **caractérisé en ce qu'**un canal d'air annulaire périphérique (16) existe sur le bord inférieur (14) des membranes (4), lié ou permettant d'être lié de façon étanche aux paires de canaux (15) et/ou **en ce que** le ou les écarteurs (9) ou au moins une partie des écarteurs (9) est(sont) situé(s) au niveau de la bordure (14) de la membrane (4).

12. Plafond à double membrane selon une des revendications précédentes, **caractérisé par** le placement des écarteurs (9) dans la zone du manchon de raccordement (7a) et/ou par le placement du manchon de raccordement (7a) dans la zone du canal d'air annulaire(16) ou d'une paire de canaux d'air (15) et/ou en ce que les écarteurs (9) et/ou les poches de fixation (13) isolent thermiquement et/ou sont fabriqués de façon isolantes et/ou en ce que les écarteurs (9) sont fabriqués en plastique et en particulier en plastique extrudé.

13. Plafond à double membrane selon une des revendications précédentes, **caractérisé en ce que** les membranes (4) et/ou les poches de fixation (13) et/ou les écarteurs (9) sont fabriqués en matière plastique renforcéeen fibres de verre (GFK), en matière plastique renforcée en fibres de carbone (KFK), en polyéthylène (PE), en polypropylène (PP), en polychlorure de vinyle (PVC), en polystyrène (PS), en polyuréthane (PU),en polyéthylène téréphtalate (PET), en polytétrafluoroéthylène (PTFE), en polymétachrylate de méthyle (PMMA) ou en une quelconque combinaison de ces matières et/ou **en ce que** le plafond à double membrane (2) possède une forme de cône ou de coupelle.

14. Conteneur de fermentation d'une installation biogaz avec un plafond à double membrane (2), **caractérisé en ce que** le plafond à double membrane (2) est configuré selon une des revendications précédentes.

15. Utilisation d'une membrane (4) destinée à un plafond à double membrane (2) en particulier en forme de cône ou de coupelle couvrant un conteneur de fermentation (1) d'une installation biogaz, **caractérisé en ce que** les membranes (4) formant le plafond à double membrane (2), en particulier en forme de cône ou de coupelle, sont fabriquées selon une des revendications 1 à 13.
